# EUROPÄISCHE PATENTANMELDUNG

(11) **EP 1 382 595 A1**
(43) Veröffentlichungstag der Anmeldung: **21.01.2004**
(21) Anmeldenummer: 03018727.2
(22) Anmeldetag: 27.01.2001
(51) Int. Cl.: C07C 227/02, B01J 25/00

(54) **Festbettraney-Kupferkatalysator zur Dehydrierung von Alkoholen**

(30) Priorität: 18.02.2000 EP 00103547
(62) Teilanmeldung aus: 01101897.5
(71) Anmelder: Degussa AG, 40474 Düsseldorf (DE)
(72) Erfinder: Ostgard, Daniel, Dr., 63801 Kleinostheim (DE); Berweiler, Monika, 63477 Maintal (DE); Seelbach, Karsten, 73432 Aalen-Waldhausen (DE)
(74) Vertreter: Weber, Wolfgang, Dr

(57) **Zusammenfassung**

Festbettraney-Kupferkatalysator bei dem die ursprüngliche Legierung mehr als 50 % Kupfer enthält, sodass der entgültige Katalysator mehr restliches Aluminium enthält als normalerweise unter denselben Aktivierungsbedingen vorhanden ist, wird als Festbettkatalysator bei der Festbettdehydrierung von Alkoholen eingesetzt.

## Beschreibung

Die Erfindung betrifft einen Festbettraney-Kupferkatalysator, ein Verfahren zu seiner Herstellung sowie ein Verfahren zur Dehydrierung von Alkoholen.

Es ist bekannt, Diethanolamin zu Iminodiessigsäure zu dehydrieren. (US 5,689,000; WO 96/01146; WO 92/06949; JP-OS 091 55 195; US 5,292,936; US 5,367,112; CA 212 10 20)

Gegenstand der Erfindung ist Festbettraney-Kupferkatalysator, welcher dadurch gekennzeichnet ist, daß er als Tabletten, Extrudate, Hohlkörper, Fasertabletten, Granulaten,gegebenenfalls gebunden auf einen Träger, und/oder Tellergranulaten hergestellt ist. Der Festbettraney-Kupferkatalysator, kann mittels einem oder mehreren Metallen aus der Gruppe Eisen und/oder Edelmetall dotiert sein. Gegebenenfalls kann er zusätzlich andere Dotierungsmetalle, z.B. Bi, Sn, Sb, Pb, Ge, Cr, Mo, Ti , Ni, Ta, Zr, V, Mn, W, Co und/oder Nb und/oder Mischungen davon, enthalten.

Das Dotierungs-Metall kann sowohl in das Kupfer reinlegiert als auch nachträglich aufgeschichtet sein.

Das erfindungsgemäße Raney-Kupfer kann die Dotierungselemente in einer Menge von 10 ppm bis 1 Gew.-% enthalten. Die Edelmetalldotierung kann 10 bis 50.000 ppm, vorzugsweise 500 bis 50.000 ppm, betragen. Die Dotierungsmetalle können aus der Gruppe Eisen sowie Palladium, Platin, Gold, Silber, Rhenium, Iridium, Ruthenium und/oder Rhodium ausgewählt werden.

Insbesondere kann für die Dotierung ein Metall aus der Gruppe Pt, Pd und/oder Fe ausgewählt sein.

Der Katalysator kann andere zusätzliche Promotoren enthalten. Die ursprünglich gebildete Legierung kann mehr als 50 % Kupfer enthalten, sodaß der endgültige Katalysator mehr restliches Aluminium enthält als normalerweise unter denselben Aktivierungsbedingungen gefunden wird.

Die ursprünglich gebildete Legierung kann einer Hitzebehandlung in Luftatmosphäre bei Temperaturen oberhalb von 500 °C vor der Aktivierung unterzogen werden.

Die ursprünglich gebildete Legierung kann mehr als 50 % Kupfer enthalten und in Luftatmosphäre einer Hitzebehandlung bei Temperaturen oberhalb 500 °C vor der Aktivierung unterzogen werden.

Die mittlere Teilchengröße des erfindungsgemäßen Festbettraney-Kupferkatalysators kann von 0.05 mm bis 20 mm betragen.

Die mittlere Teichengröße des erfindungsgemäßen Festbettraney-Kupferkatalysators ist von Bedeutung bei der Anwendung bei Oxidationsreaktionen beziehungsweise Dehydrierungsreak tionen von Alkoholen.

Das erfindungsgemäße, Festbettraney-Kupferkatalysator wird vorteilhafterweise nicht durch eine unerwünschte Vergiftung oder einen unerwüschten Abrieb desaktiviert.

Ein weiterer Gegenstand der Erfindung ist ein Verfahren zur Herstellung des erfindungsgemäßen Festbettraney-Kupferkatalysators, welches dadurch gekennzeichnet ist, daß man einen Festbett-Raney-Katalysator auf bekanntem Wege herstellt, verformt, aktiviert, mit mindestens einem Dotierungsmetall dotiert, wäscht und trocknet.

Die Dotierung mittels eines Dotiermetalls kann erfolgen, indem man den aktivierten Katalysator in einem Säulenreaktor mit Lösungskreislauf einfüllt und zu der umlaufenden Lösung die Dotiermetall-Lösung hinzugibt.

Die Verformung des Katalysators kann auf bekanntem Wege erfolgen.

In einer besonderen Ausführungsform kann man den erfindungsgemäß dotierten Katalysator zu Hohlkugeln verformen. Dabei kann man das Legierungspulver mit gegebenenfalls weiteren Bestandteilen in einer wässrigen Lösung suspendieren und diese Suspension auf leicht brennbare Kugeln zum Beispiel Polystyrolkugeln aufsprühen Dieser Beschichtungsvorgang kann gegebenenfalls wiederholt werden. Nach der Beschichtung kann jeweils in einem Luftstrom getrocknet werden.

Anschliessend werden die leicht brennbaren Kugeln herausgebrannt. Anschließend werden die erhaltenden Hohlkugeln mittels Natronlauge aktiviert und mittels Metallsalzlösung dotiert, gewaschen und getrocknet.

Ein weiterer Gegenstand der Erfindung ist ein Verfahren zur katalytische Dehydrierung von Alkoholen, welches dadurch gekennzeichnet ist, daß man als Festbettkatalysator ein mit Eisen und/oder Edelmetall, und gegebenenfalls anderen geeignete Dotierungsmetallen dotierten Festbettraney-Kupferkatalysator verwendet.

Das erfindungsgemäße Verfahren zur Dehydrierung von Alkoholen kann zur Dehydrierung von Glykolen und/oder Aminoalkoholen zu den entsprechenden Carbonyl-Verbindungen und Carbonsäuren verwendet werden. Dabei kann der Festbettkatalysator als Tabletten, Extrudate, Hohlkörper; Fasertabletten, Granulate, gebunden auf ein Träger, und Tellergranulaten eingesetzt werden.

Die Alkohole, die erfindungsgemäß dehydriert werden können, können ein- oder mehrwertige Alkohole sein. Sie können inklusive von Polyetherglykolen aliphatische, cyclische oder aromatische Verbindungen sein, die mit einer starken Base zu dem Carboxylate reagieren.

Hierbei ist es notwendig, daß der Alkohol und das resultierende Carboxylat in stark basischer Lösung stabil sind, und der Alkohol wenigstens etwas in Wasser löslich ist.

Geeignete primäre, einwertige Alkohole können umfassen:
Aliphatische Alkohole, die verzweigte, gradkettige, cyclische oder aromatische Alkohole, wie zum Beispiel Benzylalkohol, sein können, wobei diese Alkohole mit verschiedenen, basenstabilen Gruppen substituiert sein können.
Geeignete aliphatische Alkohole können Ethanol, Propanol, Butanol, Pentanol oder ähnlich sein.

Erfindungsgemäß können Glykole zu Carbonsäuren oxidiert beziehungsweise dehydriert werden.

So kann man zum Beispiel Ethylenglykol zu Glykolsäure (Monocarbonsäure) dehydrieren und durch anschliessende Umsetzung mit KOH die Dicarbonsäure Oxalsäure herstellen.

Aminoalkohole können ebenfalls mit dem erfindungsgemäß, mit Edelmetall dotierten Raney-Kupfer zu den entsprechenden Aminocarbonsäuren dehydriert werden. Die Aminoalkohole können 1 bis 50 C-Atome aufweisen.

So kann man beispielsweise
N-Methylethanolamin zu Sarkosin; THEEDA zu EDTA; Monoethanolamin zu Glycin;
Diethanolamin zu Iminodiessigsäure;
3-Amino-1-propanol zu Beta-Alanin;
2-Amino-1-butanol zu 2-Aminobuttersäure dehydrieren.

In einer Ausführungsform der Erfindung können mit dem erfindungsgemäßen Verfahren Amonoalkohole der Formel 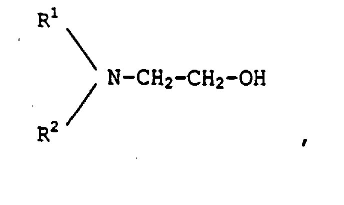 in der R¹ und R² jeweils Wasserstoff; Hydroxyethyl; - CH₂CO₂H; eine Alkylgruppe mit 1 bis 18 C-Atomen; eine Aminoalkylgruppe mit 1 bis 3 C-Atomen; eine Hydroxyalkylaminoalkyl-Gruppe mit 2 bis 3 C-Atomen sowie Phosphonomethyl bedeuten, dehydriert werden.

Die Aminoalkohole, die erfindungsgemäß eingesetzt werden können, sind bekannt. Wenn R¹ und R² gleich Wasserstoff sind, dann ist der Aminoalkohol Diethanolamin.

Wenn R¹ und R² Hydroxyethyl sind, dann ist der Aminoalkohol Triethanolamine. Die resultierenden Aminocarbonsäuresalze dieser Ausgangsaminoalkohole sollten die Salze von Glycin, Iminodiessigsäure, beziehungsweise Nitrilotriessigsäure sein. Weitere Aminoalkohole umfassen N-methylethanolamin, N,N-dimethylethanolamin, N-ethylethanolamin, N-isopropylethanolamin, N- butylethanolamin, N-nonylethanolamine, N-(2-aminoethyl)ethanolamin, N-(3-aminopropyl)ethanolamin, N,N-diethylethanolamin, N,N-dibutylethanolamin, N-methyldiethanolamin, N-ethyldiethanolamin, N-isopropyldiethanolamin, N-butyldiethanolamin, N-ethyl,N-(2-aminoethyl)-ethanolamin, N-methyl,N-(3-aminopropyl)ethanolamin, tetra(2-hydroxyethyl)ethylenediamin, und ähnliche.

Weitere Beispiele für Aminocarbonsäuresalze sind die Salze von N-methylglycin, N,N-dimethylglycin,N-ethylglycin, N-isopropylglycin, N-butylglycin, N-nonylglycin, N-(2-aminoethyl)glycin, N-3-aminopropyl)glycin, N,N-diethylglycin, N,N-dibutylglycin, N-methyliminodiessigsäure, N-ethyliminodiessigsäure, N-isopropyliminodiessigsäure, N-butyliminodiessigsäure, N-ethyl, N-(2-aminoethyl)glycin, N-methyl-N-(3-aminopropyl)glycin, Ethylenediaminetetraessigsäure, und so weiter.

R¹ oder R² können ebenfalls eine Phosphonomethyl-Gruppe sein, wobei die Ausgangsaminoverbindung N-phosphonomethylethanolamin und die resulierend Aminosäure N-phosphonomethylglycin sein können. Wenn von R¹ oder R² ein R = Phosphonomethyl und das andere R = -CH₂CH₂OH ist, wäre die resultierende Aminosäure N-phosphonomethyliminodiessigsäure, die zu N-phosphonomethylglycin auf bekanntem Wege umgewandelt werden kann. Wenn von R¹ oder R² ein R = Phosphonomethyl ist und das andere R = eine Alkylgruppe ist, wäre die resultierende Säure N-alkyl-N-phosphonomethylglycin, das zu N-phosphonomethylglycine entsprechend dem U.S. Patent 5,068,404 weiter umgewandelt werden kann.

Das erfindungsgemäße Verfahren kann bei einer Temperatur von 50 bis 250 °C, bevorzugt 80 bis 200 °C, und bei einem Druck von 0,1 bis 200 bar, bevorzugt Normaldruck bis 50 bar, durchgeführt werden.

Der Druck ist notwendig, weil die Alkohole einen hohen Dampfdruck aufweisen. Bei dem Ablassen des Wasserstoffes würde bei einem zu niedrigen Druck auch der Alkohol abgelassen werden.

Das erfindungsgemäße Verfahren weist die folgenden Vorteile auf:

Bekannte, pulverisierte Katalysatoren weisen den Nachteil auf, dass sie nur in einem diskontinuierlichen Verfahren verwendet werden können und nach der katalytischen Umsetzung aus dem Reaktionsmedium durch kostspieliges Absetzen und/oder Filtration abgetrennt werden müssen.

Die erfindungsgemäßen Festbettkatalysatoren sind geeignet für kontinuierliche Verfahren. Die Reaktionlösung kann leichter von Katalysator getrennt werden.

Die stabilisierten Katalysatoren und Katalysatoren mit keiner unaktivierten Legierung haben auch einen Vorteil in der benötigen basischeren Lösung, die man für die Alkoholdehydrierung verwenden muß. Diese Katalysatoren werden nicht während der Reaktion weiter aktiviert. Die Stabilisierung des Katalysators könnte entweder mit einem höheren Anteil von Cu-Binder, wobei der Kupferanteil 2,5 bis 70 % betragen kann, oder mit einer höheren Kalzinierungstemperatur, aber ohne der Bildung von Alpha-Aluminiumoxid, durchgeführt werden.

Die Edelmetalle, Eisen oder mit anderen Metallen dotierten Festbettraney-Kupferkatalysatoren haben weiterhin den Vorteil, daß sie einen verbesserten Widerstand gegen chemische oder mechanische Desaktivierung haben. Beispielen für die chemische Desaktivierung könnten giftige Verbindungen in den Edukt, giftige Nebenprodukte und zersetzte Verbindungen auf der katalytische Oberfläche sein.

Beispiele für die mechanische Desaktivierung könnten der Abrieb oder die Zersetzung der Formkörper sein.

### Beispiel 1 (Vergleichsbeigpiel)

Gemäß EP 0 6 48 534 A1 wird für einen Vergleichskatalysator, der aus 1.000 g Legierungspulver aus 50% Cu und 50% Al, 100 g reinem Kupferpulver (99% Kupfer, d50=21 µm) und 25 g Ethylenbis-stearoylamid besteht, unter Zugabe von etwa 150 g Wasser ein freifließendes pelletisierbares Katalysatorgemisch hergestellt. Aus diesem Gemisch werden Tabletten mit einem Durchmesser von 3 mm und einer Dicke von 3 mm gepresst. Die Formkörper werden 2 Stunden bei 700°C kalziniert. Die Tabletten werden in 20%iger Natronlauge 2 Stunden bei 40-80°C nach dem Kalzinieren aktiviert. Unter den Bedingungen des Anwendungsbeispiels braucht dieser Katalysator mehr als 7 Stunden für die Dehydrierung von 378,0 g Diethanolamin zu Iminodiessigsäure.

### Beispiel 2 (Vergleichsbeispiel)

Gemäß EP 0 6 48 534 A1 wird für einen Vergleichskatalysator, der aus 1.000 g Legierungspulver aus 50% Cu und 50% Al, 675 g reinem Kupferpulver (99% Kupfer, d50=21 µm) und 25 g Ethylenbis-stearoylamid besteht, unter Zugabe von etwa 150 g Wasser ein freifließendes pelletisierbares Katalysatorgemisch hergestellt. Aus diesem Gemisch werden Tabletten mit einem Durchmesser von 3 mm und einer Dicke von 3 mm gepresst. Die Formkörper werden 2 Stunden bei 700°C kalziniert. Die Tabletten werden in 20%iger Natronlauge 2 Stunden bei 40―80°C nach dem Kalzinieren aktiviert. Unter den Bedingungen des Anwendungsbeispiels braucht dieser Katalysator für die Dehydrierung von 189,0 g Diethanolamin zu Iminodiessigsäure 130 Minuten für den ersten Zyklus und 150 Minuten für die Zyklen 2, 3 und 4.

### Beispiel 3

Gemäß EP 0 6 48 534 A1 wird für einen Katalysator, der aus 1.000 g Legierungspulver aus 50% Cu und 50% Al, 100 g reinem Kupferpulver (99% Kupfer, d50=21 µm) und 25 g Ethylenbis-stearoylamid besteht, unter Zugabe von etwa 150 g Wasser ein freifließendes pelletisierbares Katalysatorgemisch hergestellt. Aus diesem Gemisch werden Tabletten mit einem Durchmesser von 3 mm und einer Dicke von 3 mm gepresst. Die Formkörper werden 2 Stunden bei 700°C kalziniert. Die Tabletten werden in 20%iger Natronlauge 2 Stunden bei 40-80°C nach dem Kalzinieren aktiviert. Anschließend wird Hexachloroplatin zu der Suspension des gewaschenen Katalysators gegeben. Der pH-Wert wird eingestellt und die Suspension weiter gerührt. Der dotierte Katalysator wird anschliessend gewaschen. Der Platingehalt des Katalysators ist 1%.

### Beispiel 4

Gemäß EP 0 6 48 534 A1 wird für ein Katalysator, der aus 1.000 g Legierungspulver aus 50% Cu und 50% Al, 675 g reinem Kupferpulver (99% Kupfer, d50=21 µm) und 25 g Ethylenbis-stearoylamid besteht, unter Zugabe von etwa 150 g Wasser ein freifließendes pelletisierbares Katalysatorgemisch hergestellt. Aus diesem Gemisch werden Tabletten mit einem Durchmesser von 3 mm und einer Dicke von 3 mm gepresst. Die Formkörper werden 2 Stunden bei 700°C kalziniert. Die Tabletten werden in 20%iger Natronlauge 2 Stunden bei 40-80°C nach dem Kalzinieren aktiviert. Anschließend wird Hexachloroplatin zu der Suspension des gewaschenen Katalysators gegeben. Der pH-Wert wird eingestellt und die Suspension weiter gerührt. Der dotierte Katalysator wird anschliessend gewaschen. Der Platingehalt des Katalysators ist 1%.

### Beispiel 5

Gemäß EP 0 6 48 534 A1 wird für einen Katalysator, der aus 1.000 g Legierungspulver aus 50% Cu und 50% Al, 100 g reinem Kupferpulver (99% Kupfer, d50=21 µm) und 25 g Ethylenbis-stearoylamid besteht, unter Zugabe von etwa 150 g Wasser ein freifließendes pelletisierbares Katalysatorgemisch hergestellt. Aus diesem Gemisch werden Tabletten mit einem Durchmesser von 3 mm und einer Dicke von 3 mm gepresst. Die Formkörper werden 2 Stunden bei 700°C kalziniert. Die Tabletten werden in 20%iger Natronlauge 2 Stunden bei 40-80°C nach dem Kalzinieren aktiviert. Anschließend wird Eisen III Chlorid zu der Suspension des gewaschenen Katalysators gegeben. Der pH-Wert wird eingestellt und die Suspension weiter gerührt. Der dotierte Katalysator wird anschliessend gewaschen. Der Eisengehalt des Katalysators ist 3%.

### Beispiel 6

Gemäß EP 0 6 48 534 A1 wird für einen Katalysator, der aus 1.000 g Legierungspulver aus 50% Cu und 50% Al, 675 g reinem Kupferpulver (99% Kupfer, d50=21 µm) und 25 g Ethylenbis-stearoylamid besteht, unter Zugabe von etwa 150 g Wasser ein freifließendes pelletisierbares Katalysatorgemisch hergestellt. Aus diesem Gemisch werden Tabletten mit einem Durchmesser von 3 mm und einer Dicke von 3 mm gepresst. Die Formkörper werden 2 Stunden bei 700°C kalziniert. Die Tabletten werden in 20%iger Natronlauge 2 Stunden bei 40-80°C nach dem Kalzinieren aktiviert. Anschließend wird Eisen III Chlorid zu der Suspension des gewaschenen Katalysators gegeben. Der pH-Wert wird eingestellt und die Suspension weiter gerührt. Der dotierte Katalysator wird anschliessend gewaschen. Der Eisengehalt des Katalysators ist 3%.

### Beispiel 7

Durch Suspendieren von 800 g einer Legierung aus 50% Cu/50% Al und 104 g Kupferpulver in 1.000 ml wässeriger Lösung mit einem Gehalt an 5 Gew.% Polyvinylalkohol und 1,25 Gew.% Glycerin wird eine Beschichtungslösung hergestellt. Diese Suspension wird sodann auf 2.000 ml Polystyrolkugeln im Bereich von 4 bis 5 mm aufgesprüht, während diese in nach oben strömender. Luft suspendiert sind. Nach Beschichten der Polystyrolkugeln mit der zuvor genannten Lösung werden die Kugeln in aufwärts strömender Luft bei Temperaturen von bis zu 80°C getrocknet (Höhere Temperaturen können auch angewandt werden). Diese getrockneten, beschichteten Polystyrolkugeln haben eine Schüttdichte von 0,26 g/ml, und die Hälfte dieser Kugeln wird mit einer Legierungslösung weiter beschichtet. Die Lösung für die zweite Schicht besteht aus 800 g einer Legierung aus 50% Cu/50% Al und 104 g Kupferpulver, das in 1.000 ml wässeriger Lösung mit einem Gehalt an 5 Gew.% Polyvinylalkohol und 1,25 Gew.% Glycerin suspendiert ist. Diese Suspension wird sodann auf 1.000 ml der mit Cu/Al vorbeschichteten und getrockneten, zuvor erwähnten Polystyrolkugeln aufgesprüht, während diese in einem aufwärts gerichteten Luftstrom suspendiert sind. Nach Beschichten der Polystyrolkugeln mit der zuvor genannten Lösung werden die Kugeln in aufwärts strömender Luft bei Temperaturen von bis zu 80°C getrocknet (Es können auch höhere Temperaturen angewandt werden). Die getrockneten, beschichteten Kugeln werden sodann in einem gesteuerten Stickstoff/Luftstrom bei 550°C zum Herausbrennen des Styropors erwärmt und um das Kupfer und die Legierungsteilchen zusammenzusintern. Die Hohlkugeln werden sodann in einer 20 gew.%igen Natronlauge bei 80°C 1,5 Stunden aktiviert. Die erhaltenen aktivierten Hohlkugeln haben einen durchschnittlichen Durchmesser von 6 mm, eine Manteldicke im Bereich von 600 bis 700 µ und eine Schüttdichte von 0,60 g/ml. Wie aus der Entwicklung von Wasserstoffblasen visuell ersichtlich ist, hat der Katalysator ein großes Reservoir an aktivem Wasserstoff.

### Beispiel 8

Durch Suspendieren von 800 g einer Legierung aus 50% Cu/50% Al und 104 g Kupferpulver in 1.000 ml wässeriger Lösung mit einem Gehalt an 5 Gew.% Polyvinylalkohol und 1,25 Gew.% Glycerin wirde eine Beschichtungslösung hergestellt. Diese Suspension wird sodann auf 2.000 ml Polystyrolkugeln im Bereich von 4 bis 5 mm aufgesprüht, während diese in nach oben strömender Luft suspendiert sind. Nach Beschichten der Polystyrolkugeln mit der zuvor genannten Lösung werden die Kugeln in aufwärts strömender Luft bei Temperaturen von bis zu 80°C getrocknet (Höhere Temperaturen können auch angewandt werden). Diese getrockneten, beschichteten Polystyrolkugeln haben eine Schüttdichte von 0,26 g/ml, und die Hälfte dieser Kugeln wird mit einer Legierungslösung weiter beschichtet. Die Lösung für die zweite Schicht besteht aus 800 g einer Legierung aus 50% Cu/50% Al und 104 g Kupferpulver, das in 1.000 ml wässeriger Lösung mit einem Gehalt an 5 Gew.% Polyvinylalkohol und 1,25 Gew.% Glycerin suspendiert ist. Diese Suspension wird sodann auf 1.000 ml der mit Cu/Al vorbeschichteten und getrockneten, zuvor erwähnten Polystyrolkugeln aufgesprüht, während diese in einem aufwärts gerichteten Luftstrom suspendiert wird. Nach Beschichten der Polystyrolkugeln mit der zuvor genannten Lösung werden die Kugeln in aufwärts strömender Luft bei Temperaturen von bis zu 80°C getrocknet (Es können auch höhere Temperaturen angewandt werden). Die getrockneten, beschichteten Kugeln werden sodann in einem gesteuerten Stickstoff/Luftstrom bei 550°C zum Herausbrennen des Styropors erwärmt und um das Kupfer und die Legierungsteilchen zusammenzusintern. Die Hohlkugeln werden sodann in einer 20 gew.%igen Natronlauge bei 80°C 1,5 Stunden aktiviert. Die erhaltenen aktivierten Hohlkugeln haben einen durchschnittlichen Durchmesser von 6 mm, eine Manteldicke im Bereich von 600 bis 700 p und eine Schüttdichte von 0,60 g/ml. Wie aus der Entwicklung von Wasserstoffblasen visuell ersichtlich ist, hat der Katalysator ein großes Reservoir an aktivem Wasserstoff. Anschließend wird Hexachloroplatin zu der Suspension des gewaschenen Katalysators gegeben. Der pH-Wert wird eingestellt und die Suspension weiter gerührt. Der dotierte Katalysator wird anschliessend gewaschen. Der Platingehalt des Katalysators ist 1%.

### Beispiel 9

Durch Suspendieren von 800 g einer Legierung aus 50% Cu/50% Al und 104 g Kupferpulver in 1.000 ml wässeriger Lösung mit einem Gehalt an 5 Gew.% Polyvinylalkohol und 1,25 Gew.% Glycerin wird eine Beschichtungslösung hergestellt. Diese Suspension wird sodann auf 2.000 ml Polystyrolkugeln im Bereich von 4 bis 5 mm aufgesprüht, während diese in nach oben strömender Luft suspendiert sind. Nach Beschichten der Polystyrolkugeln mit der zuvor genannten Lösung werden die Kugeln in aufwärts strömender Luft bei Temperaturen von bis zu 80°C getrocknet (Höhere Temperaturen können auch angewandt werden). Diese getrockneten, beschichteten Polystyrolkugeln haben eine Schüttdichte von 0,26 g/ml, und die Hälfte dieser Kugeln sind mit einer Legierungslösung weiter beschichtet. Die Lösung für die zweite Schicht besteht aus 800 g einer Legierung aus 50% Cu/50% Al und 104 g Kupferpulver, das in 1.000 ml wässeriger Lösung mit einem Gehalt an 5 Gew.% Polyvinylalkohol und 1,25 Gew.% Glycerin suspendiert ist. Diese Suspension wird sodann auf 1.000 ml der mit Cu/Al vorbeschichteten und getrockneten, zuvor erwähnten Polystyrolkugeln aufgesprüht, während diese in einem aufwärts gerichteten Luftstrom suspendiert sind. Nach Beschichten der Polystyrolkugeln mit der zuvor genannten Lösung werden die Kugeln in aufwärts strömender Luft bei Temperaturen von bis zu 80°C getrocknet (Es können auch höhere Temperaturen angewandt werden). Die getrockneten, beschichteten Kugeln werden sodann in einem gesteuerten Stickstoff/Luftstrom bei 550°C zum Herausbrennen des Styropors erwärmt und um das Kupfer und die Legierungsteilchen zusammenzusintern. Die Hohlkugeln werden sodann in einer 20 gew.%igen Natronlauge bei 80°C 1,5 Stunden aktiviert. Die erhaltenen aktivierten Hohlkugeln haben einen durchschnittlichen Durchmesser von 6 mm, eine Manteldicke im Bereich von 600 bis 700 µ und eine Schüttdichte von 0,60 g/ml. Wie aus der Entwicklung von Wasserstoffblasen visuell ersichtlich ist, hat der Katalysator ein großes Reservoir an aktivem Wasserstoff. Anschließend wird Eisen III Chlorid zu der Suspension des gewaschenen Katalysators gegeben. Der pH-Wert wird eingestellt und die Suspension weiter gerührt. Der dotierte Katalysator wird anschliessend gewaschen. Der Eisengehalt des Katalysators ist 3%.

### Beispiel 10

### Herstellung von Iminodiessigsäure mit einem Festbettraney-Kupferkatalysator.

Das Beispiel illustriert die Umsetzung von Diethanolamin (DEA) zum Natrium-Salz der Iminodiessigsäure (IDA) mit den festbettraney-Kupferkatalysators.

Die Versuche werden in einem Festbett-Rohrreaktor mit Flüssigkeitskreislauf durchgeführt. Im Festbett-Rohrreaktor wird folgender Ansatz vorgelegt:
100 - 400 g Diethanolamin (3 mol)
266 - 1064 g wässrige NaOH-Lösung (30 Gew.-%). Die Verhältnis zum Diaethanolamin ist 2,66
200 g erfindungsgemäßer Festbettraney-Kupferkatalysatoren
186 - 744 g H₂O, ulträschall-entgast. Die Verhältnis zum Diethanolamin ist 1,86

Der Festbett-Rohrreaktor wird mit Stickstoff auf 10 bar aufgedrückt und auf Reaktionstemperatur gebracht (TR=170°C). Nach Anspringen der Reaktion wird der entstehende Wasserstoff abgelassen, wobei die freigesetzte Menge über eine Trockengas-Uhr bestimmt wird. Die Reaktion wird nach einer Dauer von 5 h abgebrochen und der Autoklav abgekühlt. Währen des Reaktion werden Proben von der Reaktionlösung genommen und durch gaschromatographische Auftrennung analysiert.

Der eingesetzte Katalysator kann ohne nennenswerten Aktivitätsverlust mehrfach rezykliert werden.

## Patentansprüche

1. Festbettraney-Kupferkatalysator bei dem die ursprüngliche Legierung mehr als 50 % Kupfer enthält, sodaß der entgültige Katalysator mehr restliches Aluminium enthält als normalerweise unter denselben Aktivierungsbedingen vorhanden ist.

2. Verfahren zur katalytischen Dehydrierung von Alkoholen zu ihren entsprechenden Carbonyl-Verbindungen und Carbonsäuren, wobei ein Festbettraney-Kupferkatalysator gemäß dem Anspruchen 1 eingesetzt wird.
